# FASCICULE DE BREVET EUROPEEN

(11) **EP 1 579 841 B1**
(45) Date de publication et mention de la délivrance du brevet: **14.11.2012**
(21) Numéro de dépôt: 05290411.7
(22) Date de dépôt: 23.02.2005
(51) Int. Cl.: A61K 8/891, A61Q 1/12

(54) **Composition cosmétique comprenant des particules concaves**
Kosmetische Zusammensetzung enthaltend konkave Partikel
Cosmetic composition comprising concave particles

(30) Priorité: 22.03.2004 FR 0450563
(43) Date de publication de la demande: 28.09.2005
(73) Titulaire: L'Oréal, 75008 Paris (FR)
(72) Inventeur: Dumousseaux, Christophe, Shinjukn Tokyo (JP)
(74) Mandataire: Boulard, Denis

(56) Documents cités:
- EP-A- 0 445 785
- US-B1- 6 461 595
- PATENT ABSTRACTS OF JAPAN vol. 2000, no. 10, 17 novembre 2000 (2000-11-17) & JP 2000 191789 A (TAKEMOTO OIL & FAT CO LTD), 11 juillet 2000 (2000-07-11)
- PATENT ABSTRACTS OF JAPAN vol. 2003, no. 09, 3 septembre 2003 (2003-09-03) & JP 2003 128788 A (TAKEMOTO OIL & FAT CO LTD), 8 mai 2003 (2003-05-08)

## Description

La présente invention a pour objet une composition cosmétique, en particulier sous forme de poudre compacte, comprenant des particules concaves et des particules en forme de plaquettes. L'invention a également pour objet un procédé de maquillage ou de soin des matières kératiniques d'être humain, comme la peau, les cheveux, les ongles, et plus particulièrement la peau, comprenant l'application de la composition sur les matières kératiniques.

La composition selon l'invention peut être une composition de maquillage ou de soin de la peau et peut se présenter sous la forme d'un fard à joues, un fard à paupières, une poudre pour le visage, un fond de teint, un produit anticerne, un produit de maquillage du corps, d'un produit de soin du visage, d'un produit de soin du corps, d'un produit antisolaire. Plus spécialement, l'invention porte sur une composition de fond de teint.

Les poudres de maquillage comprennent généralement, d'une part, une phase pulvérulente comportant notamment des pigments et des charges et d'autre part, une phase grasse au titre de liant comprenant des corps gras, destinée à conférer au produit fini une certaine densité, à donner une douceur et une propriété émolliente au produit de maquillage et à favoriser son adhérence sur la peau.

Certaines compositions de maquillage telles que les fonds de teint, les fards à paupières ou les fards à joues se présentent sous forme de poudre compacte, comprenant généralement une phase grasse, appelée liant, et une phase pulvérulente comprenant notamment des pigments et/ou des nacres et/ou des charges.

L'élaboration des poudres compactes soulève de nombreuses difficultés car la composition finale doit être suffisamment homogène et compacte pour éviter une fragmentation provoquée notamment par les chocs. La poudre compacte doit également présenter une bonne aptitude au prélèvement pour permettre à l'utilisatrice d'appliquer la composition sur les matières kératiniques, notamment sur la peau.

Par ailleurs, les nacres sont couramment employées comme matière colorantes dans les produits de maquillage. Les nacres se présentent généralement sous forme de plaquettes et, du fait de leur forme et de leur taille, provoquent un frein lors de l'application du maquillage sur la peau rendant ainsi l'application du produit désagréable pour l'utilisatrice et ne favorisant le bon étalement du produit sur la peau de manière uniforme.

Le document EP-A-0 445 785 divulgue des particules de sulfate de baryum, et des compositions les comprenant

Le but de la présente invention est donc de disposer d'une composition de maquillage contenant des particules en forme de plaquettes et s'étalant facilement, avec un bon glissant, sur les matières kératiniques, en particulier sur la peau, et, lorsqu'elle se présente sous forme de poudre compacte, pouvant se déliter facilement au doigt ou à l'aide d'une éponge.

Les inventeurs ont découvert qu'une telle composition est obtenue en associant avec les particules en forme de plaquettes des particules concaves sous forme de portions de sphères creuses constituées d'un matériau organosiliconé.
La composition présente alors un bon glissant facilitant une bonne répartition du produit sur la surface de la peau et permettant ainsi d'obtenir un maquillage réparti de manière homogène sur la peau. De plus, lorsque la composition est sous la forme de poudre compacte, présente de bonnes propriétés de délitage permettant à l'utilisatrice de prendre facilement au doigt ou à l'éponge la quantité nécessaire de produit pour se maquiller.

De façon plus précise, l'invention a pour objet une composition cosmétique, notamment sous forme de poudre compacte, comprenant une phase pulvérulente, la phase pulvérulente comprenant des particules concaves sous forme de portions de sphères creuses constituées d'un matériau organosiliconé et des particules en forme de plaquettes.

L'invention a également pour objet un procédé cosmétique de maquillage ou de traitement non thérapeutique des matières kératiniques, notamment de la peau, comprenant l'application sur les matières kératiniques, notamment sur la peau, d'une composition telle que définie précédemment.

La composition selon l'invention comprend des particules concaves. Ces particules ont donc une surface présentant un arrondi intérieur.

Les particules concaves sont des particules de portions de sphères creuses constituées d'un matériau organosiliconé.

Lesdites particules concaves ont avantageusement un diamètre moyen allant de 0,05 µm à 10 µm.

Les portions de sphères creuses utilisées dans la composition selon l'invention peuvent avoir la forme de sphères creuses tronquées, présentant un seul orifice communiquant avec leur cavité centrale, et ayant une section transversale en forme de fer à cheval ou d'arceau.

Le matériau organosiliconé est un polysiloxane réticulé de structure tridimensionnelle ; il comprend de préférence, voire est constitué de, des motifs de formule (I) : SiO₂ et de formule (II) : R¹SiO_{1,5}
dans lesquels R¹ désigne un groupe organique ayant un atome de carbone directement relié à l'atome de silicium. Le groupe organique peut être un groupe organique réactif, un groupe organique non réactif, et de préférence un groupe organique non réactif.

Le groupe organique non réactif peut être un groupe alkyle en C₁-C₄, notamment un groupe méthyle, éthyle, propyle, butyle, ou un groupe phényle, et de préférence un groupe méthyle.

Le groupe organique réactif peut être un groupe époxy, un groupe (méth)acryloxy, un groupe alkényle, un groupe mercaptoalkyle, aminoalkyle, halogénoalkyle, un groupe glycéroxy, un groupe uréïdo, un groupe cyano. De préférence, le groupe organique réactif peut être un groupe époxy, un groupe (méth)acryloxy, un groupe alkényle, un groupe mercaptoalkyle, aminoalkyle. Le groupe organique réactif comprend généralement de 2 à 6 atomes de carbone, notamment de 2 à 4 atomes de carbone.

Comme groupe époxy, on peut citer un groupe 2-glycidoxyéthyl, un groupe 3-glycidoxypropyl, un groupe 2-(3,4-époxycyclohexyl)propyl.
Comme groupe (méth)acryloxy, on peut citer un groupe 3-méthacryloxypropyl, un groupe 3-acryloxypropyl.
Comme groupe alkényle, on peut citer un groupe vinyl, allyl, isopropényl.
Comme groupe mercaptoalkyle, on peut citer un groupe mercaptopropyl, mercaptoéthyl.
Comme groupe aminoalkyle, on peut citer un groupe 3-(2-aminoéthyl)aminopropyl, un groupe 3-aminopropyl, un groupe N,N-diméthylaminopropyl.
Comme groupe halogénoalkyle, on peut citer un groupe 3-chloropropyl, un groupe trifluoropropyl.
Comme groupe glycéroxy, on peut citer un groupe 3-glycéroxypropyl, un groupe 2-glycéroxyéthyl.
Comme groupe uréido, on peut citer un groupe 2-uréidoéthyl.
Comme groupe cyano, on peut citer un groupe cyanopropyl, cyanoéthyl.

De préférence, dans le motif de formule (II), R¹ désigne un groupe méthyle.

Avantageusement, le matériau organosiliconé comprend les motifs (I) et (II) selon un rapport molaire motif (I) / motif (II) allant de 30/70 à 50/50, de préférence allant de 35/65 à 45/55.

Les particules d'organosilicone peuvent être notamment susceptibles d'être obtenues selon un procédé comprenant :
(a) l'introduction dans un milieu aqueux, en présence d'au moins un catalyseur d'hydrolyse, et éventuellement d'au moins un tensioactif, d'un composé (III) de formule SiX₄ et d'un composé (IV) de formule RSiY₃, où X et Y désignent indépendamment l'un de l'autre un groupe alcoxy en C₁-C₄, un groupe alcoxyéthoxy renfermant un groupement alcoxy en C₁-C₄, un groupe acyloxy en C₂-C₄, un groupe N,N-dialkylamino renfermant un groupement alkyle en C₁-C₄, un groupe hydroxyle, un atome d'halogène ou un atome d'hydrogène, et R désigne un groupe organique comportant un atome de carbone directement relié à l'atome de silicium ; et
(b) la mise en contact du mélange résultant de l'étape (a) avec une solution aqueuse renfermant au moins un catalyseur de polymérisation et éventuellement au moins un tensioactif, à une température comprise entre 30 et 85°C, pendant au moins deux heures.

L'étape (a) correspond à une réaction d'hydrolyse et l'étape (b) correspond à une réaction de condensation.

Dans l'étape (a), le rapport molaire du composé (III) au composé (IV) va habituellement de 30/70 à 50/50, avantageusement de 35/65 à 45/45, et est préférentiellement de 40/60. Le rapport en poids de l'eau au total des composés (III) et (IV) va de préférence de 10/90 à 70/30. L'ordre d'introduction des composés (III) et (IV) dépend généralement de leur vitesse d'hydrolyse. La température de la réaction d'hydrolyse va généralement de 0 à 40 °C et ne dépasse habituellement pas 30°C pour éviter une condensation prématurée des composés.

### Pour les groupements X et Y des composés (III) et (IV) :

Comme groupe alcoxy en C₁-C₄, on peut citer les groupes méthoxy, éthoxy ;
Comme groupe alkoxyéthoxy renfermant un groupe alcoxy en C₁-C₄, on peut citer les groupes méthoxyéthoxy, butoxyéthoxy ;
Comme groupe alcoxy en C₂-C₄, on peut citer les groupes acétoxy, propioxy ;
Comme groupe N,N-dilakylamino renfermant un groupement alkyle en C₁-C₄, on peut citer les groupes diméthylamino, diéthylamino ;
Comme atome d'halogène, on peut citer les atomes de chlore, de brome.

Comme composés de formule (III), on peut citer le tétraméthoxysilane, le tétraéthoxysilane, le tétrabutoxysilane, le triméthoxyéthoxysilane, le tributoxyéthoxysilane, le tétraacétoxysilane, le tétrapropioxysilane, le tétraacétoxysilane, le tétra(diméthylamino)silane, le tétra(diéthylamino)silane, le silane tétraol, le chlorosilane triol, le dichlorodisilanol, le tétrachlorosilane, le chlorotrihydrogénosilane. De préférence, le composé de formule (III) est choisi parmi le tétraméthoxysilane, le tétraéthoxysilane, le tétrabutoxysilane, et leurs mélanges.

Le composé de formule (III) conduit après la réaction de polymérisation à la formation des motifs de formule (I).

Le composé de formule (IV) conduit après la réaction de polymérisation à la formation des motifs de formule (II).

Le groupe R dans le composé de formule (IV) a la signification telle que décrite pour le groupe R¹ pour le composé de formule (II).

Comme exemple de composés de formule (IV) comportant un groupe R organique non réactif, on peut citer le méthyltriméthoxysilane, l'éthyltriéthoxysilane, le propyltributoxysilane, le butyltributoxysilane, le phényltriméthoxyéthoxysilane, le méthyl tributoxyethoxysilane, le méthyltriacétoxysilane, le méthyltripropioxysilane, le méthyltriacétoxysilane, le méthyltri(diméthylamino)silane, le méthyltri(diéthylamino)silane, le méthylsilane triol, le méthylchlorodisilanol, le méthyltrichlorosilane, le méthyltrihydrogénosilane.

Comme exemple de composés de formule (IV) comportant un groupe R organique réactif, on peut citer :
les silanes ayant un groupe époxy comme le 3-glycidoxypropyl triméthoxysilane, le 3-glycidoxypropyl triéthoxysilane, le 2-(3,4-époxycyclohexyl)éthyl triméthoxysilane, le 3-glycidoxypropylméthyl diméthoxysilane, le 3-glycidoxypropylméthyl diméthoxysilane, le 2-glycidoxyéthylméthyldiméthoxysilane, le 3-glycidoxypropyl diméthylméthoxysilane, le 2-glycidoxyéthyl diméthylméthoxysilane ;
les silanes ayant un groupe (méth)acryloxy comme le 3-méthacryloxypropyl triméthoxysilane, le 3-acryloxypropyl triméthoxysilane ;
les silanes ayant un groupe alkényle comme le vinyl triméthoxysilane, l'allyl triméthoxysilane, l'isopropényl triméthoxysilane ;
les silanes ayant un groupe mercapto comme le mercaptopropyl triméthoxysilane, le mercaptoéthyl triméthoxysilane ;
les silanes ayant un groupe aminoalkyle comme le 3-aminopropyl triméthoxysilane, le 3-(2-aminoéthyl)aminopropyl triméthoxysilane, le N,N-diméthylaminopropyl triméthoxysilane, le N,N-diméthylaminoéthyl triméthoxysilane ;
les silanes ayant un groupe halogénoalkyl comme le 3-chloropropyl triméthoxysilane, le trifluoropropyl triméthoxysilane ;
les silanes ayant un groupe glycéroxy comme le 3-glycéroxypropyl triméthoxysilane, le di(3-glycéroxypropyl)diméthoxysilane ;
les silanes ayant un groupe uréido comme le 3-uréïdopropyl triméthoxysilane, le 3-uréïdopropyl méthyldiméthoxysilane, le 3-uréïdopropyl diméthylméthoxysilane ;
les silanes ayant un groupe cyano comme le cyanopropyl triméthoxysilane, le cyanopropyl méthyldiméthoxysilane, le cyanopropyl diméthylméthoxysilane.

De préférence, le composé de formule (IV) comportant un groupe R organique réactif est choisi parmi les silanes ayant un groupe époxy, les silanes ayant un groupe (méth)acryloxy, les silanes ayant un groupe alkényle, les silanes ayant un groupe mercapto, les silanes ayant un groupe aminoalkyle.

Des exemples de composés (III) et (IV) préférés pour la mise en oeuvre de cette invention sont respectivement le tétraéthoxysilane et le méthyltriméthoxysilane.

Comme catalyseurs d'hydrolyse et de polymérisation, on peut utiliser indépendamment des catalyseurs basiques - tels que l'hydroxyde de sodium, l'hydroxyde de potassium, le carbonate de sodium, l'hydrogénocarbonate de sodium, ou des amines (telles qu'ammoniaque, triméthylamine, triéthylamine, hydroxyde de tétraméthylammonium) - ou des catalyseurs acides, choisis parmi les acides organiques - tels que l'acide citrique, l'acide acétique, l'acide méthanesulfonique, l'acide p-toulène sulfonique, l'acide dodécylbenzènesulfonique, l'acide dodécylsulfonique - ou minéraux - tels que l'acide chlorhydrique, l'acide sulfurique, l'acide phosphorique. Lorsqu'il est présent, le tensioactif utilisé est de préférence un tensioactif non ionique ou anionique ou un mélange des deux. Le dodécylbenzènesulfonate de sodium peut être utilisé comme tensioactif anionique. La fin de l'hydrolyse est marquée par la disparition des produits (III) et (IV), insolubles dans l'eau, et l'obtention d'une couche liquide homogène.

L'étape (b) de condensation peut utiliser le même catalyseur que l'étape d'hydrolyse ou un autre catalyseur choisi parmi ceux mentionnés précédemment.

A l'issue de ce procédé, on obtient une suspension dans l'eau de fines particules organosiliconées qui peuvent éventuellement être ensuite séparées de leur milieu. Le procédé décrit ci-dessus peut donc comprendre une étape supplémentaire de filtration, par exemple sur filtre à membrane, du produit résultant de l'étape (b), suivie éventuellement d'une étape de centrifugation du filtrat destinée à séparer les particules du milieu liquide, puis d'une étape de séchage des particules. D'autres méthodes de séparation peuvent bien entendu être employées.

Les particules obtenues (ou les sphères) ont de préférence un diamètre moyen allant de 0,05 à 10 µm.

La forme des portions de sphères creuses obtenues selon le procédé ci-dessus, ainsi que leurs dimensions, dépendront notamment du mode de mise en contact des produits à l'étape (b).

Un pH plutôt basique et une introduction à froid du catalyseur de polymérisation dans le mélange issu de l'étape (a) conduira à des portions de sphères creuses en forme de "bols" à fond arrondi, tandis qu'un pH plutôt acide, et une introduction goutte-à-goutte du mélange issu de l'étape (a) dans le catalyseur de polymérisation chaud, conduira à des portions de sphères creuses ayant une section transversale en forme de "fer à cheval".

Selon un mode de réalisation préféré de l'invention, on utilise des portions de sphères creuses en forme de "bols". Celles-ci peuvent être obtenues comme décrit dans la demande JP-2003 128 788.
Des portions de sphères creuses en forme de fer à cheval sont aussi décrites dans la demande JP-A-2000-191789.

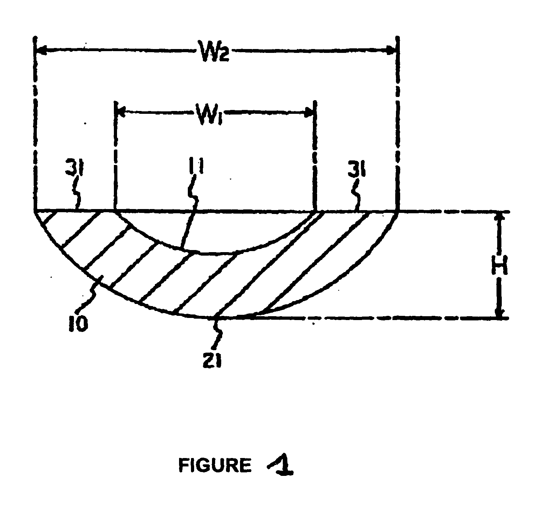

La figure annexée illustre une particule concave de portions de sphères en forme de bol en coupe transversale.

Comme il ressort de cette figure, ces portions concaves sont formées (en coupe longitudinale) d'un petit arc interne (11), d'un grand arc externe (21) et de segments (31) qui relient les extrémités des arcs respectifs, la largeur (W1) entre les deux extrémités du petit arc interne (11) allant de 0,01 à 8 µm, de préférence de 0,02 à 6 µm en moyenne, la largeur (W2) entre les eux extrémités du grand arc externe (21) allant de 0,05 à 10 µm, de préférence de 0,06 à 8 µm en moyenne et la hauteur (H) du grand arc externe (21) allant de 0,015 à 8 µm, de préférence de 0,03 à 6 µm en moyenne.

Les dimensions mentionnées ci-dessus sont obtenues en calculant la moyenne des dimensions de cent particules choisies sur une image obtenue au microscope électronique à balayage.

Comme particules concaves de portions de sphères utilisables selon l'invention, on peut citer :
les particules constituées de l'organosilicone réticulé TAK-110 (polymère réticulé méthylsilanol/silicate) de la société TAKEMOTO OIL & FAT , en forme de bol , de largeur 2,5 µm, de hauteur 1,2 µm et d'épaisseur 150 nm (particules vendues sous la dénomination NLK-506 par la société Takemoto Oil & Fat) ;
les particules constituées de l'organosilicone réticulé TAK-110 (polymère réticulé méthylsilanol/silicate) de la société TAKEMOTO OIL & FAT , en forme de bol , de largeur 2,5 µm, de hauteur 1,5 µm et d'épaisseur 350 nm ;
les particules constituées de l'organosilicone réticulé TAK-110 (polymère réticulé méthylsilanol/silicate) de la société TAKEMOTO OIL & FAT , en forme de bol , de largeur 0,7 µm, de hauteur 0,35 µm et d'épaisseur 100 nm ;
les particules constituées de l'organosilicone réticulé TAK-110 (polymère réticulé méthylsilanol/silicate) de la société TAKEMOTO OIL & FAT , en forme de bol , de largeur 7,5 µm, de hauteur 3,5 µm et d'épaisseur 200 nm.

Les particules concaves, notamment les particules de portions de sphères creuses, peuvent être présentes dans composition selon l'invention, notamment dans la poudre compacte, en une teneur allant de 0,01 % à 50 % en poids, par rapport au poids total de la composition, de préférence allant de 0,1 % à 30 % en poids, et préférentiellement allant de 1 % à 15% en poids.

On entend par « plaquette » selon la présente demande, des particules dont la forme est caractérisée par trois dimensions : une longueur, une largeur et une hauteur, appelée aussi épaisseur, dont le rapport de la plus grande dimension à l'épaisseur est supérieur ou égal à 5.

Au sens de la présente invention, on entend par « plus grande dimension des plaquettes », le diamètre de la sphère dans laquelle s'inscrit ladite plaquette.

Les particules en forme de plaquette peuvent être de forme parallélépipédique (surface rectangulaire ou carrée), discoïdale (surface circulaire) ou ellipsoïdale (surface ovale).

Les plaquettes peuvent être choisies parmi les plaquettes de matériau minéraux. En particulier, les plaquettes peuvent être choisies parmi les plaquettes de mica, de séricite, de verre (notamment borosilicate de calcium), de silice, d'oxyde d'aluminium, de sulfate de baryum.

Les plaquettes peuvent être traitées en surface, notamment recouvertes par une couche de métal ou d'oxyde métallique.
Comme métal, on peut citer l'argent, l'aluminium, le chrome, le nickel, le molybdène, l'or, le cuivre, l'étain, le magnésium et leurs mélanges (alliages). On utilise de préférence l'argent, le chrome, le nickel, le molybdène, et leurs mélanges.

Comme oxyde métallique, on peut citer le dioxyde de titane, les oxydes de fer, les oxyde de zinc , l'oxyde de chrome, et de préférence le dioxyde de titane.

Comme plaquettes, on peut notamment utiliser :
- les plaquettes de mica vendues sous les dénominations "Mica Concord 1000" par la société Sciama, "Cardre Mica 8" par la socité Cardre, "Mica 40" par la société Eckart, "PDM-20 L", "PDM-40 L" par la société Topy ;
- les plaquettes de séricite vendues sous les dénominations "Sericite FS", "Sericite FSE" par la société Sanshin Mining, "NAI-S-100", "Sericite S-152" par la société Miyoshi, "Synthetice FNK-100" par la société Topy ;
- les plaquettes d'oxydes d'aluminium vendues sous la dénomination "Luxelen FAO" par la société Asahi ;
- les plaquettes de silice vendues sous les dénominations "TSG 30A Flake", "PTSG 30 A Flake", "Silica Flake SG" par la société Nippon Sheet Glass ;
- les plaquettes de sulfate de baryum vendues sous la dénomination "Flake shaped Baryum sulfate" par la société Sakai Chemical ;

- les plaquettes de mica recouvertes de dioxyde de titane vendues sous les dénominations "Flamenco", 'Duochrome", "Cloisonne" par la société Engelhard, "Timiron" par la société Merck ;
- les plaquettes de mica synthétique recouvertes de dioxyde de titane vendues sous les dénominations "Prominence" par la société Nihon Koken, "Sunshine" par la société Sun Chemical ;
- les plaquettes d'oxyde d'aluminium recouvertes de dioxyde de titane commercialisées sous les dénominations "Xirona silver", "Xirallic" par la société Merck ;
- les plaquettes de silice recouvertes de dioxyde de titane vendues sous les dénominations "Colorstream" et "Xirona" par la société Merck ;
- les plaquettes de verre recouvertes d'une couche métallique, on peut utiliser par exemples les particules recouvertes d'argent vendues sous les dénominations Microglass Metashine REFSX 2025 PS, GF 2140 par la société TOYAL, les particules recouvertes d'alliage nickel/chrome/molybdène vendues sous les dénominations Crystal star GF 550, GF 2525 par la société TOYAL ;
- les plaquettes de verre recouvertes de dioxyde de titane vendues sous la dénomination "Reflecks" par la société Engelhard ;
les plaquettes de talc recouvertes de dioxyde de titane vendues sous la dénomination "Silseem" par la société Nihon Koken.

Les plaquettes peuvent être également traitées en surface par un agent de traitement hydrophobe.

L'agent de traitement hydrophobe peut être choisi parmi les silicones comme les méthicones, les diméthicones, les perfluoroalkylsilanes ; les acides gras comme l'acide stéarique ; les savons métalliques comme le dimyristate d'aluminium, le sel d'aluminium du glutamate de suif hydrogéné, les perfluoroalkyl phosphates, les perfluoroalkyl si lanes, les perfluoroalkyl silazanes, les polyoxydes d'hexafluoropropylène, les polyorganosiloxanes comprenant des groupes perfluoroalkylles perfluoropolyéthers, les acides aminés ; les acides aminés N-acylés ou leurs sels ; la lécithine, le trisostéaryle titanate d'isopropyle, et leurs mélanges.
Les acides aminés N-acylés peuvent comprendre un groupe acyle ayant de 8 à 22 atomes de carbones, comme par exemple un groupe 2-éthyl hexanoyle, caproyle, lauroyle, myristoyle, palmitoyle, stéaroyle, cocoyle. Les sels de ces composés peuvent être les sels d'aluminium, de magnésium, de calcium, de zirconium, de zin, de sodium, de potassium. L'acide aminé peut être par exemple la lysine, l'acide glutamique, l'alanine

Le terme alkyl mentionné dans les composés cités précédemment désigne notamment un groupe alkyle ayant de 1 à 30 atomes de carbone, de préférence ayant de 5 à 16 atomes de carbone.
Des pigments traités hydrophobes sont notamment décrits dans la demande EP-A-1086683.

Les plaquettes peuvent être également des fibres plates.

Par "fibre", il faut comprendre un objet de longueur L et de diamètre D tel que L soit très supérieur à D, D étant le diamètre du cercle dans lequel s'inscrit la section (section transversale) de la fibre. En particulier, le rapport UD (ou facteur de forme) est choisi dans la gamme allant de 5 à 2 500, de préférence de 5 à 500, et mieux de 5 à 150.

Par "fibre plate", on entend une fibre dont la section transversale (section perpendiculaire à l'axe de la direction de la longueur de la fibre) présente une plus grande longueur L1 et une plus petite longueur L2 (L2 correspond à l'épaisseur de la fibre) telle que L1/L2 (le rapport L1/L2 est encore appelé facteur d'aplatissement) est supérieur ou égal à 4, de préférence supérieur à 7. Notamment, L1/L2 va de 4 à 15, de préférence de 6 à 12 , et mieux de 7 à 10. Ainsi, la section transversale de la fibre présente une forme plate. Avantageusement, la plus grande longueur L1 et la plus petite longueur L2 définissent respectivement des axes X1, X2 tels que l'axe X1 est sensiblement perpendiculaire à l'axe X2.

La plus grande longueur L1 correspond au diamètre D de la fibre tel que mentionné précédemment. Ainsi, les fibres plates peuvent se présenter sous la forme de ruban ou de tagliatelle.

Les fibres plates peuvent notamment présenter une section transversale de forme sensiblement rectangulaire, ovoïdale ou ellipsoïdale.

Les fibres utilisables dans la composition de l'invention peuvent être des fibres d'origine synthétique, organique, et plus particulièrement des fibres de polymère synthétique. Elles peuvent être courtes ou longues, unitaires (ou monofilament) ou organisées par exemple tressées (ou multi-filaments), creuses ou pleines, de préférence pleine. Lorsque les fibres sont des fibres multifilaments, chaque filament peut être de composition chimique différente et présenter une couleur différente : on obtient ainsi des fibres multifilaments présentant des couleurs différentes. En particulier, leurs extrémités sont épointées et/ou polies pour éviter de se blesser. Avantageusement, les fibres plates sont insolubles dans l'eau.
La fibre plate peut être torsadée le long de l'axe de la longueur L de la fibre. Lorsque la fibre plate n'est pas torsadée, elle présente une couleur dans un certain angle de vue, en dehors de cet angle la fibre est transparente ou de couleur blanche. La fibre plate torsadée quant à elle présente une couleur quel que soit l'angle d'observation.

En particulier, les fibres ont une longueur allant de 1 µm à 10 mm, de préférence de 0,1 mm à 5 mm et mieux de 0,3 mm à 3,5 mm. Leur section transversale (section plate) peut être comprise dans un cercle de diamètre allant de 2 nm à 500 µm, de préférence allant de 100 nm à 100 µm et mieux de 1 µm à 70 µm. Le poids ou titre des fibres est souvent donné en denier ou décitex et représente le poids en gramme pour 9 km de fil. De préférence, les fibres selon l'invention ont un titre choisi dans la gamme allant de 0,15 à 30 deniers et mieux de 0,18 à 18 deniers.

Les fibres peuvent être des fibres de rayonne, de polyamide (Nylon®), de viscose, d'acétate notamment d'acétate de rayonne, de poly-(p-phénylène-téréphtalamide) (ou d'aramide) notamment de Kevlar®, de polymère acrylique notamment de polyméthacrylate de méthyle ou de polyméthacrylate de 2-hydroxyéthyle, de polyoléfine et notamment de polyéthylène ou de polypropylène, de polytétrafluoroéthylène (comme le Téflon®), de polychlorure de vinyle ou de vinylidène, de polyfluorure de vinylidène, d'alcool polyvinylique, de polyacrylonitrile, de polyuréthane, de polyesters comme les polyéthylène téréphtalates, les polyéthylène naphtalates, de polycarbonate.

Des fibres plates sont notamment décrites dans la demande WO-A-02/41851 dont le contenu est incorporé dans la présente demande à titre de référence.

En particulier, on peut utiliser les fibres plates à structure multicouche comprenant des couches alternées de polymères choisis parmi les polyesters, les polymères acryliques, les polyamides, tellles que celles décrites dans les documents EP-A-6921217, EP-A-686858 et US-A-5472798. De telles fibres sont vendues sous les dénominations "Morphotex", « Teijin Tetron Morphotex » par la société TEIJIN.

Les plaquettes peuvent être présentes dans la composition selon l'invention en une teneur allant de 1 à 99 % en poids, par rapport au poids total de la composition, de préférence allant de 5 % à 90 %, et préférentiellement allant de 10 % à 80 % en poids.

De préférence, les particules en forme de plaquettes et les particules concaves sont présentes dans la composition selon l'invention en une teneur telle que le rapport pondéral particules en forme de plaquettes / particules concaves va de 1 à 100, de préférence va de 2 à 75, et préférentiellement va de 5 à 50.

Avantageusement, les particules concaves de portions de sphères creuses sont présentes dans la composition selon l'invention en une teneur allant de 1 % à 50 % en poids, par rapport au poids total des particules concaves et des particules en forme de plaquettes, de préférence allant de 2 % à 45 % en poids, et préférentiellement allant de 5 à 40 % en poids.

La composition selon l'invention peut comprendre une matière colorante additionnelles, différentes des plaquettes décrites précédemment, et pouvant notamment être choisie parmi les pigments, les paillettes.

Par pigments, il faut comprendre des particules de toute forme, blanches ou colorées, minérales ou organiques, insolubles dans le milieu physiologique, destinées à colorer la composition.

Les pigments peuvent être blancs ou colorés, minéraux et/ou organiques. On peut citer, parmi les pigments minéraux, le dioxyde de titane, éventuellement traité en surface, les oxydes de zirconium ou de cérium, ainsi que les oxydes de zinc, de fer (noir, jaune ou rouge) ou de chrome, le violet de manganèse, le bleu outremer, l'hydrate de chrome et le bleu ferrique, les poudres métalliques comme la poudre d'aluminium, la poudre de cuivre.

Parmi les pigments organiques, on peut citer le noir de carbone, les pigments de type D & C, et les laques à base de carmin de cochenille, de baryum, strontium, calcium, aluminium.

Les pigments peuvent être présents dans la composition en un teneur allant de 0,1 % à 15 % en poids, par rapport au poids de la composition, de préférence allant de 0,5 % à 12 % en poids, et préférentiellement allant de 1 % à 10 % en poids.

La composition selon l'invention, notamment la poudre compacte, peut comprendre avantageusement une phase grasse liquide (liquide à la température ambiante (25 °C)), appelée généralement liant. Cette phase grasse liquide peut comprendre une huile utilisée habituellement dans les poudres compactes.
L'huile peut être choisie parmi les huiles utilisées classiquement comme liant dans les poudres compactes. Parmi les huiles additionnelles utilisables, on peut citer l'huile de vison, l'huile de tortue, l'huile de soja, l'huile de pépins de raison, l'huile de sésame, l'huile de maïs, l'huile de colza, l'huile de tournesol, l'huile de coton, l'huile d'avocat, l'huile d'olive, l'huile de ricin, l'huile de jojoba, l'huile d'arachide; les huiles d'hydrocarbures, telles que les huiles de paraffine, le squalane, la vaseline, le polydécène ; les esters gras, tels que le myristate d'isopropyle, le palmitate d'isopropyle, le stéarate de butyle, le stéarate d'isodécyle, le laurate d'hexyle, l'isononanoate d'isononyle, le palmitate de 2-éthyl-hexyle, le laurate de 2-hexyl-décyle, le palmitate de 2-octyl-décyle, le myristate ou le lactate de 2-octyl-dodécyle, le succinate de 2-diéthyl-hexyle, le malate de diisostéaryle, le triisostéarate de glycérine ou de diglycérine; les huiles de silicone telles que les polyméthylsiloxanes, les polyméthylphénylsiloxanes, les polysiloxanes modifiés par des acides gras, des alcools gras ou des polyoxyalkylènes, les silicones fluorées, les huiles perfluorées ; l'acide oléique, l'acide linoléique, l'acide linolénique ou l'acide isostéarique; les alcools gras supérieurs tels que le cétanol ou l'alcool oléique.

La phase grasse liquide peut être présente en une teneur allant de 0,1 % à 13 % en poids, par rapport au poids total de la composition, de préférence allant de 0,1 % à 10 % en poids, et préférentiellement allant de 0,1 % à 8 % en poids.

La composition selon l'invention, notamment la poudre compacte, peut également comprendre des charges additionnelles différentes des plaquettes décrites précédemment.
Par charges, il faut comprendre des particules, incolores ou blanches, minérales ou de synthèse, insolubles dans le milieu de la composition quelle que soit la température à laquelle la composition est fabriquée.

Les charges additionnelles peuvent être minérales ou organiques, notamment de forme sphériques ou oblongues, quelle que soit la forme cristallographique ( par exemple feuillet, cubique, hexagonale, orthorombique, etc) . On peut citer le talc, le mica, la silice, le kaolin, les poudres de polyamide (Nylon®), de poly-β-alanine et de polyéthylène, les poudres de polyuéthane, les poudres de polymères de tétrafluoroéthylène (Téflon®), la lauroyl-lysine, l'amidon, le nitrure de bore, les microsphères creuses polymériques telles que celles de chlorure de polyvinylidène/acrylonitrile comme l'Expancel® (Nobel Industrie), de copolymères d'acide acrylique, les microbilles de résine de silicone (Tospearls® de Toshiba, par exemple), les particules de polyorganosiloxanes élastomères, le carbonate de calcium précipité, le carbonate et l'hydro-carbonate de magnésium, l'hydroxyapatite, les microsphères de silice creuses, les microcapsules de verre ou de céramique, les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium.

Les charges additionnelles peuvent être présentes dans la composition en une teneur allant de 0,1 % à 90 % en poids, par rapport au poids total de la composition, de préférence allant de 1 % à 80 % en poids, et préférentiellement allant de 5 % à 70 % en poids.

La composition peut contenir d'autres ingrédients cosmétiques usuels pouvant être choisis notamment parmi les antioxydants, les parfums, les conservateurs, les neutralisants, les tensioactifs, les cires, les filtres solaires, les vitamines, les hydratants, les composés auto-bronzants, les actifs antirides.

Bien entendu l'homme du métier veillera à choisir ce ou ces éventuels composés complémentaires, et/ou leur quantité, de manière telle que les propriétés avantageuses de la composition selon l'invention ne soient pas, ou substantiellement pas, altérées par l'adjonction envisagée.

La composition selon l'invention peut être une composition anhydre, c'est-à-dire une composition contenant moins de 2 % en poids d'eau, voire moins de 0,5 % d'eau, notamment exempte d'eau, l'eau n'étant pas ajoutée lors de la préparation de la composition mais correspondant à l'eau résiduelle apportée par les ingrédients mélangés.

La composition peut être préparée en mélangeant les ingrédients de la phase pulvérulentes (particules d'organosilicone, charges et pigments) puis en ajoutant la phase grasse sous agitation, le mélange étant ensuite broyé, tamisé, puis versé dans une coupelle et compacté.

Le mélange de la phase pulvérulente et de la phase grasse broyé et tamisé est compacté à l'aide d'une presse, notamment en appliquant une pression allant de 0,5 MPa à 10 MPa, et de préférence allant de 1 MPa à 5 MPa.
La composition ainsi obtenue se présente sous forme de poudre compacte.

L'invention est illustrée plus en détails par les exemples décrits ci-après.

### Exemple 1 :

On a préparé une poudre compacte ayant la composition suivante :

Les teneurs sont exprimées en % en poids.

| | |
|---|---|
| Talc | 39,9 |
| Plaquettes de séricite (3) | 33,25 |
| Particules(1) hémisphériques en forme de bol de largeur moyenne 2,5 µm, d'épaisseur 0,15 µm et de hauteur 1,2 µm, constituées de l'organosilicone* | 5 |
| TAK-110 de la société Takemoto Oil & Fat | |
| Dioxyde de titane | 5,2 |
| Plaquettes de mica (2) | 4,75 |
| Oxyde de fer | 2,1 |
| Stéarate de zinc | 0,95 |
| Paraffine liquide | 3,8 |
| Phényl triméthicone | 4,75 |
| Conservateurs | 0,3 |

| | |
|---|---|
| * polymère réticulé méthylsilanol/silicate | |
| (1) particules vendues sous la dénomination NLK-506 par la société Takemoto Oil & Fat | |
| (2) vendues sous la dénomination "Mica Concord 1000" par la société Sciama. | |
| (3) vendues sous la dénomination "NAI-S-100" par la société Miyoshi | |

La composition a été préparée en mélangeant l'ensemble des poudres puis en y ajoutant le liant (huiles), ce mélange étant ensuite broyé et tamisé jusqu'à obtention d'un mélange homogène. 14 g de ce mélange est placé dans une coupelle puis pressé sous une pression de 2 MPa.

On a ainsi obtenu une poudre compacte qui se délite facilement à l'aide d'une éponge et la poudre prélevée glisse et s'étale bien sur la peau, avec douceur, et permet d'obtenir un maquillage très couvrant.

### Exemple 2 :

On a préparé 6 mélanges de particules hémisphériques en forme de bol identiques à celles utilisées dans l'exemple 1 précédent et de plaquettes de mica vendues sous la dénomination "Mica Concord 1000" par la société Sciama en des proportions pondérales variées. On a mesuré pour chaque mélange le coefficient de friction à l'aide d'un tribomètre (appareil de mesure du coefficient de friction) vendu sous la référence commerciale "KES-E Friction tester" par la société Katotech , équipé d'une sonde ayant une masse de 25 g et en réglant la vitesse de déplacement de la sonde à une vitesse de 1 mm/s. Pour chaque mélange, on a étalé une couche du mélange d'une épaisseur d'environ 1 mm sur une lame de verre recouverte d'un scotch double face vendu sous la dénomination "Nistack NW-20 par la société Nichiban. Puis on effectue la mesure du coefficient de friction de chaque mélange avec l'appareil : la sonde de l'appareil se déplace en venant frotter dans la couche du mélange déposée sur la lame de verre. La valeur moyenne du coefficient de friction dynamique est calculée comme la moyenne des valeurs mesurées entre le deuxième et le cinquième passage successif de la sonde sur un même échantillon.

On a obtenu les résultats suivants :

| Exemples | Plaquettes de mica (en % poids) | Particules d'organosilicone (% en poids) | Valeur moyenne du coefficient de friction |
|---|---|---|---|
| A | 100 | 0 | 0.77 |
| B | 95 | 5 | 0.66 |
| C | 90 | 10 | 0.63 |
| D | 80 | 20 | 0.59 |
| E | 60 | 40 | 0.56 |
| F | 0 | 100 | 0.68 |

On a constaté que les mélanges B, C, D et E contenant de 5 à 40 % de particules d'organosilicone présentent un coefficient de friction plus faible que celui des mélanges A et F. Ainsi, le mélange de plaquettes de mica et de particules d'organosilicone présente des propriétés de glissant plus importantes que celles de chacune des particules utilisées seules.

## Revendications

1. Composition cosmétique comprenant une phase pulvérulente, la phase pulvérulente comprenant des particules concaves sous forme de portions de sphères creuses et des particules en forme de plaquettes, **caractérisée par le fait que** les particules sous forme de portions de sphères creuses sont constituées d'un matériau organosiliconé.

2. Composition selon la revendication précédente, **caractérisée par le fait que** lesdites particules ont un diamètre moyen compris entre 0,05 et 10 µm.

3. Composition selon la revendication 1 ou 2, **caractérisée par le fait que** les particules de portions de sphères creuses ont une section transversale en forme de fer à cheval ou d'arceau.

4. Composition selon la revendication 1, 2 ou 3, **caractérisée par le fait que** le matériau organosiliconé est un polysiloxane réticulé de structure tridimensionnelle comprenant, ou constitué de, des motifs de formule (I): SiO₂ et de formule (II) : R¹SiO₁₅ dans lesquelles R¹ désigne un groupe organique ayant un atome de carbone directement relié à l'atome de silicium.

5. Composition selon la revendication précédente, **caractérisée par le fait que** le groupe organique est un groupe organique réactif, un groupe organique non réactif, et de préférence un groupe organique non réactif.

6. Composition selon la revendication 5, **caractérisée par le fait que** le groupe organique non réactif peut être un groupe alkyle en C₁-C₄, ou un groupe phényle.

7. Composition selon la revendication 5 ou 6, **caractérisée par le fait que** le groupe organique non réactif est un groupe méthyle.

8. Composition selon la revendication 5, **caractérisée par le fait que** le groupe organique réactif est choisi parmi un groupe époxy, un groupe (méth)acryloxy, un groupe alkényle, un groupe mercaptoalkyle, aminoalkyle, halogénoalkyle, un groupe glycéroxy, un groupe uréido, un groupe cyano.

9. Composition selon la revendication 5 ou 8, **caractérisée par le fait que** le groupe organique réactif est choisi parmi un groupe époxy, un groupe (méth)acryloxy, un groupe alkényle, un groupe mercaptoalkyle, aminoalkyle.

10. Composition selon la revendication 4, **caractérisée par le fait que** R¹ désigne un groupe méthyle.

11. Composition selon rune quelconque des revendications précédentes, **caractérisée par le fait que** le matériau organosiliconé comprend les motifs (I) et (II) selon un rapport molaire motif (I)/ 1 motif (II) allant de 30/70 à 50/50, de préférence allant de 35/65 à 45/55.

12. Composition selon l'une quelconque des revendications précédentes, **caractérisée par le fait que** les particules d'organosilicone sont susceptibles d'être obtenues selon un procédé comprenant :
(a) l'introduction dans un milieu aqueux, en présence d'au moins un catalyseur d'hydrolyse, et éventuellement d'au moins un tensioactif, d'un composé (III) de formule SiX₄ et d'un composé (IV) de formule RSiY_{3,} où X et Y désignent indépendamment run de l'autre un groupe alcoxy en C₁-C₄, un groupe alcoxyéthoxy renfermant un groupement alcoxy en C₁-C₄, un groupe acyloxy en C₂-C₄, un groupe N,N-dialkylamino renfermant un groupement alkyle en C₁-C₄, un groupe hydroxyle, un atome d'halogène ou un atome d'hydrogène, et R désigne un groupe organique comportant un atome de carbone directement relié à l'atome de silicium ; et
(b) la mise en contact du mélange résultant de l'étape (a) avec une solution aqueuse renfermant au moins un catalyseur de polymérisation et éventuellement au moins un tensioactif, à une température comprise entre 30 et 85°C, pendant au moins deux heures.

13. Composition selon la revendication précédente, **caractérisée par le fait que** dans l'étape (a), le rapport molaire du composé (III) au composé (IV) va de 30/70 à 50/50, de préférence va de 35/65 à 45/45, et préférentiellement est de 40/60.

14. Composition selon la revendication 12 ou 13, **caractérisée par le fait que** le rapport en poids de l'eau au total des composés (III) et (IV) va de 10/90 à 70/30 dans l'étape (a).

15. Composition selon la revendication 12, **caractérisée par le fait que** le groupe organique est un groupe organique réactif, un groupe organique non réactif, et de préférence un groupe organique non réactif.

16. Composition selon la revendication 12, **caractérisée par le fait que** le groupe organique non réactif peut être un groupe alkyle en C₁-C₄, ou un groupe phényle.

17. Composition selon la revendication 12 ou 16, **caractérisée par le fait que** le groupe organique non réactif est un groupe méthyle.

18. Composition selon la revendication 15, **caractérisée par le fait que** le groupe organique réactif est choisi parmi un groupe époxy, un groupe (méth)acryloxy, un groupe alkényle, un groupe mercaptoalkyle, aminoalkyle, halogénoalkyle, un groupe glycéroxy, un groupe uréido, un groupe cyano.

19. Composition selon la revendication 15 ou 18, **caractérisée par le fait que** le groupe organique réactif est choisi parmi un groupe époxy, un groupe (méth)acryloxy, un groupe alkényle, un groupe mercaptoalkyle, aminoalkyle.

20. Composition selon la revendication 12, **caractérisée par le fait que** R¹ désigne un groupe méthyle.

21. Composition selon l'une quelconque des revendications 12 à 20, **caractérisée par le fait que** les catalyseurs d'hydrolyse et de polymérisation sont indépendamment choisis parmi l'hydroxyde de sodium, l'hydroxyde de potassium, le carbonate de sodium, l'hydrogénocarbonate de sodium, l'ammoniaque, la triméthylamine, la triéthylamine, l'hydroxyde de tétraméthylammonium, l'acide citrique, l'acide acétique, l'acide méthanesulfonique, l'acide p-toluène sulfonique, l'acide dodécylbenzènesulfonique, l'acide dodécylsulfonique , l'acide chlorhydrique, l'acide sulfurique, l'acide phosphorique.

22. Composition selon l'une quelconque des revendications précédentes, **caractérisée par le fait que** les particules concaves sont formées (en coupe longitudinale) d'un petit arc interne (11), d'un grand arc externe (21) et de segments (31) qui relient les extrémités des arcs respectifs, la largeur (W1) entre les deux extrémités du petit arc interne (11) allant de 0,01 à 8 µm, de préférence de 0,02 à 6 µm en moyenne, la largeur (W2) entre les deux extrémités du grand arc externe (21) allant de 0,05 à 10 µm, de préférence de 0,06 à 8 µm en moyenne et la hauteur (H) du grand arc externe (21) allant de 0,015 à 8 µm, de préférence de 0,03 à 6 µm en moyenne.

23. Composition selon l'une quelconque des revendications précédentes, **caractérisée par le fait que** les particules concaves sont présentes en une teneur allant de 0,01 % à 50 % en poids, par rapport au poids total de la composition, de préférence allant de 0,1 % à 30 % en poids, et préférentiellement allant de 1 % à 15 % en poids.

24. Composition selon l'une quelconque des revendications précédentes, **caractérisée par le fait que** les particules en forme de plaquettes sont des particules ayant une plus grande dimension et une épaisseur telles que le rapport de la plus grande dimension à l'épaisseur est supérieur ou égal à 5.

25. Composition selon l'une quelconque des revendications précédentes, **caractérisée par le fait que** les plaquettes sont choisies parmi les plaquettes de matériau minéraux.

26. Composition selon l'une quelconque des revendications précédentes, **caractérisée par le fait que** les plaquettes sont choisies parmi les plaquettes de mica, de séricite, de verre, de silice, d'oxyde d'aluminium, de sulfate de baryum.

27. Composition selon l'une quelconque des revendications précédentes, **caractérisée par le fait que** les plaquettes sont recouvertes par une couche de métal ou d'oxyde métallique.

28. Composition selon la revendication précédente, **caractérisée par le fait que** le métal, est choisi parmi l'argent, l'aluminium, le chrome, le nickel, le molybdène, l'or, le cuivre, l'étain, le magnésium et leurs mélanges.

29. Composition selon la revendication 27, **caractérisée par le fait que** l'oxyde métallique, est choisi parmi le dioxyde de titane, les oxydes de fer, les oxyde de zinc, l'oxyde de chrome, et leurs mélanges.

30. Composition selon la revendication 27, **caractérisée par le fait que** l'oxyde métallique est le dioxyde de titane.

31. Composition selon l'une quelconque des revendications 1 à 23, **caractérisée par le fait que** les plaquettes sont des fibres plates.

32. Composition selon la revendication précédente, **caractérisée par le fait que** les fibres plates ont une longueur L et un diamètre D tel que UD est choisi dans la gamme allant de 5 à 2 500, de préférence de 5 à 500, et mieux de 5 à 150.

33. Composition selon la revendication 31 ou 32, **caractérisée par le fait que** les fibres ont une section transversale comprise dans un cercle de diamètre allant de 2 nm à 500 µm, de préférence allant de 100 nm à 100 µm et mieux de 1 µm à 70 µm.

34. Composition selon l'une quelconque des revendications 31 à 33, **caractérisée par le fait que** les fibres plates ont une longueur L allant de 1 µm à 10 mm, de préférence de 0,1 mm à 5 mm et mieux de 0,3 mm à 3,5 mm.

35. Composition selon l'une quelconque des revendications 31 à 34, **caractérisée par le fait que** les fibres plates ont une section transversale présentant une plus grande longueur L1 et une plus petite longueur L2 telles que L1/L2 est supérieur à 4, de préférence supérieur à 7.

36. Composition selon la revendication précédente, **caractérisée par le fait que** L1/L2 va de 4 à 15, de préférence de 6 à 12, et mieux de 7 à 10.

37. Composition selon l'une quelconque des revendications 31 à 36, **caractérisée par le fait que** les fibres plates ont une section transversale de forme rectangulaire, ovoïdale ou ellipsoïdale.

38. Composition selon l'une quelconque des revendications 31 à 37, **caractérisée par le fait que** les fibres plates se présentent sous forme de ruban ou de tagliatelle.

39. Composition selon l'une quelconque des revendications 31 à 38, **caractérisée par le fait que** les fibres plates ont un titre choisi dans la gamme allant de 0,15 à 30 deniers et mieux de 0,18 à 18 deniers.

40. Composition selon l'une quelconque des revendications 31 à 39, **caractérisée par le fait que** les fibres plates sont des fibres de polymères.

41. Composition selon l'une quelconque des revendications 31 à 40, **caractérisée par le fait que** les fibres plates sont choisies parmi les fibres de rayonne, de polyamide, de viscose, d'acétate notamment d'acétate de rayonne, de poly-(p-phénylène-téréphtatamide), de polymère acrylique, notamment de polyméthacrylate de méthyle ou de polyméthacrylate de 2-hydroxyéthyle, de polyoléfine et notamment de polyéthylène ou de polypropylène, de polytétrafluoroéthylène, de polychlorure de vinyle ou de vinylidène, de polyfluorure de vinylidène, d'alcool polyvinylique, de polyacrylonitrile, de polyuréthane, de polyesters comme les polyéthylène téréphtalates, les polyéthylène naphtalates, de polycarbonate.

42. Composition selon l'une quelconque des revendications 31 à 41, **caractérisée par le fait que** les fibres plates sont choisies parmi les fibres à structure multicouche comprenant des couches alternées de polymères choisis parmi les polyesters, les polymères acryliques, les polyamides.

43. Composition selon l'une quelconque des revendications précédentes, **caractérisée par le fait que** les particules en forme de plaquettes sont présentes en une teneur allant de 1 à 99 % en poids, par rapport au poids total de la composition, de préférence allant de 5 % à 90 %, et préférentiellement allant de 10 % à 80 % en poids.

44. Composition selon l'une quelconque des revendications précédentes, **caractérisée par le fait que** les particules en forme de plaquettes et les particules concaves sont présentes en une teneur telle que le rapport pondérai particules en forme de plaquettes / particules concaves va de 1 à 100, de préférence va de 2 à 75, et préférentiellement va de 5 à 50.

45. Composition selon l'une quelconque des revendications précédentes, **caractérisée par le fait que** les particules concaves sont présentes en une teneur allant de 1% à 50 % en poids, par rapport au poids total des particules concaves et des particules en forme de plaquettes, de préférence allant de 2 % à 45 % en poids, et préférentiellement allant de 5 à 40 % en poids.

46. Composition selon l'une quelconque des revendications précédentes, **caractérisée par le fait qu'**elle comprend une matière colorante pulvérulente additionnelle différentes desdites plaquettes et choisie parmi les pigments et les paillettes.

47. Composition selon la revendication précédente, **caractérisée par le fait que** les pigments sont choisis parmi le dioxyde de titane, l'oxyde de zirconium, l'oxyde de cérium, les oxydes de zinc, les oxydes de fer, l'oxyde de chrome, le violet de manganèse, le bleu outremer, l'hydrate de chrome, le bleu ferrique, la poudre d'aluminium, la poudre de cuivre, le noir de carbone, les pigments de type D & C, les laques.

48. Composition selon la revendication 46 ou 47, **caractérisée par le fait que** les pigments sont présents en une teneur allant de 0,1 % à 15 % en poids, par rapport au poids total de la composition, de préférence allant de 0,5 % à 12 % en poids, et préférentiellement allant de 1 % à 10 % en poids.

49. Composition selon l'une quelconque des revendications précédentes, **caractérisée par le fait qu'**elle comprend une charge additionnelle différentes desdites plaquettes.

50. Composition selon la revendication précédente, **caractérisée par le fait que** la charge additionnelle est choisie parmi le talc, le mica, la silice, le kaolin, les poudres de polyamide, les poudres de poly-β-alanine, les poudres de polyéthylène, les poudres de polyuéthane, les poudres de polymères de tétrafluoroéthylène, la lauroyl-lysine, l'amidon, le nitrure de bore, les microsphères creuses polymériques, les microbilies de résine de silicone, les particules de polyorganosiloxanes élastomères, le carbonate de calcium précipité, le carbonate et l'hydro-carbonate de magnésium, l'hydroxyapatite, les microsphères de silice creuses, les microcapsules de verre ou de céramique, les savons métalliques dérivés d'acides organiques carboxyliques ayant de 8 à 22 atomes de carbone.

51. Composition selon la revendication 49 ou 50, **caractérisée par le fait que** les charges additionnelles sont présentes en une teneur allant de 0,1 % à 90 % en poids, par rapport au poids total de la composition, de préférence allant de 1 % à 80 % en poids, et préférentiellement allant de 5 % à 70 % en poids.

52. Composition selon l'une quelconque des revendications précédentes, **caractérisée par le fait qu'**elle comprend une phase grasse liquide.

53. Composition selon la revendication précédente, **caractérisée par le fait que** la phase grasse liquide comprend une huile choisie parmi l'huile de vison, l'huile de tortue, l'huile de soja, l'huile de pépins de raison, l'huile de sésame, l'huile de maïs, l'huile de colza, l'huile de tournesol, l'huile de coton, l'huile d'avocat, l'huile d'olive, l'huile de ricin, l'huile de jojoba, l'huile d'arachide, les huiles de paraffine, le squalane, la vaseline, le polydécène, le myristate d'isopropyle, le palmitate d'isopropyle, le stéarate de butyle, le stéarate d'isodécyle, le laurate d'hexyle, l'isononanoate d'isononyte, le palmitate de 2-éthyl-hexyle, le laurate de 2-hexyl-décyle, le palmitate de 2-octyl-décyle, le myristate ou le lactate de 2-octyl-dodécyle, le succinate de 2-diéthyl-hexyle, le malate de diisostéaryle, le triisostéarate de glycérine ou de diglycérine, les huiles de silicone, les silicones fluorées, les huiles perfluorées, l'acide oléique, l'acide linoléique, l'acide linolénique, le cétanol, l'alcool oléique.

54. Composition selon la revendication 52 ou 53, **caractérisée par le fait que** la phase grasse liquide est présente en une teneur allant de 0,1% à 13 % en poids, par rapport au poids total de la composition, de préférence allant de 0,1 % à 10 % en poids, et préférentiellement allant de 0,1 % à 8 % en poids.

55. Composition selon l'une quelconque des revendications précédentes, **caractérisée par le fait qu'**elle comprend un ingrédient cosmétique choisi parmi les cires, les conservateurs, les actifs cosmétiques, les agents hydratants, les filtres UV, les épaississantes, l'eau, les tensioactifs, les parfums.

56. Composition selon l'une quelconque des revendications précédentes, **caractérisée par le fait qu'**elle se présente sous forme de poudre compacte.

57. Composition selon l'une quelconque des revendications précédentes, **caractérisée par le fait que** la composition est sous forme d'un fard à joues, un fard à paupières, une poudre pour le visage, un fond de teint, un produit anticerne, un produit de maquillage du corps, un produit de soin pour le visage, un produit de soin du corps, un produit antisolaire.

58. Procédé cosmétique de maquillage ou de traitement non thérapeutique des matières kératiniques, notamment de la peau, comprenant l'application sur les matières kératiniques, notamment sur la peau, d'une composition selon l'une quelconque des revendications précédentes.

## Claims

1. Cosmetic composition comprising a pulverulent phase, the pulverulent phase comprising concave particles in the form of portions of hollow spheres and particles with the shape of platelets, **characterized in that** the particles in the form of portions of hollow spheres are composed of an organosilicone material.

2. Composition according to the preceding claim, **characterized in that** the said particles have a mean diameter of between 0.05 µm and 10 µm.

3. Composition according to Claim 1 or 2, **characterized in that** the particles of portions of hollow spheres have a transverse cross section with the shape of a horseshoe or arch.

4. Composition according to Claim 1, 2 or 3, **characterized in that** the organosilicone material is a crosslinked polysiloxane with a three-dimensional structure comprising or composed of units of formula (I): SiO₂, and of formula (II): R¹SiO_{1.5},
in which R¹ denotes an organic group having a carbon atom directly connected to the silicon atom.

5. Composition according to the preceding claim, **characterized in that** the organic group is a reactive organic group or an unreactive organic group and preferably an unreactive organic group.

6. Composition according to Claim 5, **characterized in that** the unreactive organic group can be a C₁-C₄ alkyl group or a phenyl group.

7. Composition according to Claim 5 or 6, **characterized in that** the unreactive organic group is a methyl group.

8. Composition according to Claim 5, **characterized in that** the reactive organic group is chosen from an epoxy group, a (meth)acryloyloxy group, an alkenyl group, a mercaptoalkyl, aminoalkyl or haloalkyl group, a glyceroxy group, a ureido group or a cyano group.

9. Composition according to Claim 5 or 8, **characterized in that** the reactive organic group is chosen from an epoxy group, a (meth)acryloyloxy group, an alkenyl group or a mercaptoalkyl or aminoalkyl group.

10. Composition according to Claim 4, **characterized in that** R¹ denotes a methyl group.

11. Composition according to any one of the preceding claims, **characterized in that** the organosilicone material comprises the units (I) and (II) according to a unit (I)/unit (II) molar ratio ranging from 30/70 to 50/50, preferably ranging from 35/65 to 45/55.

12. Composition according to any one of the preceding claims, **characterized in that** the organosilicone particles are capable of being obtained according to a process comprising:
(a) the introduction into an aqueous medium, in the presence of at least one hydrolysis catalyst and optionally of at least one surfactant, of a compound (III) of formula SiX₄ and of a compound (IV) of formula RSiY₃, where X and Y denote, independently of one another, a C₁-C₄ alkoxy group, an alkoxyethoxy group including a C₁-C₄ alkoxy group, a C₂-C₄ acyloxy group, an N,N-dialkylamino group including a C₁-C₄ alkyl group, a hydroxyl group, a halogen atom or a hydrogen atom and R denotes an organic group comprising a carbon atom connected directly to the silicon atom; and
(b) the operation in which the mixture resulting from stage (a) is brought into contact with an aqueous solution including at least one polymerization catalyst and optionally at least one surfactant, at a temperature of between 30 and 85°C, for at least two hours.

13. Composition according to the preceding claim, **characterized in that**, in stage (a), the molar ratio of the compound (III) to the compound (IV) ranges from 30/70 to 50/50, preferably ranges from 35/65 to 45/45, and is preferentially 40/60.

14. Composition according to Claim 12 or 13, **characterized in that** the ratio by weight of the water to the total of the compounds (III) and (IV) ranges from 10/90 to 70/30 in stage (a).

15. Composition according to Claim 12, **characterized in that** the organic group is a reactive organic group or an unreactive organic group and preferably an unreactive organic group.

16. Composition according to Claim 12, **characterized in that** the unreactive organic group can be a C₁-C₄ alkyl group or a phenyl group.

17. Composition according to Claim 12 or 16, **characterized in that** the unreactive organic group is a methyl group.

18. Composition according to Claim 15, **characterized in that** the reactive organic group is chosen from an epoxy group, a (meth)acryloyloxy group, an alkenyl group, a mercaptoalkyl, aminoalkyl or haloalkyl group, a glyceroxy group, a ureido group or a cyano group.

19. Composition according to Claim 15 or 18, **characterized in that** the reactive organic group is chosen from an epoxy group, a (meth)acryloyloxy group, an alkenyl group or a mercaptoalkyl or aminoalkyl group.

20. Composition according to Claim 12, **characterized in that** R denotes a methyl group.

21. Composition according to any one of Claims 12 to 20, **characterized in that** the hydrolysis and polymerization catalysts are chosen independently from sodium hydroxide, potassium hydroxide, sodium carbonate, sodium hydrogencarbonate, ammonia, trimethylamine, triethylamine, tetramethylammonium hydroxide, citric acid, acetic acid, methanesulphonic acid, p-toluenesulphonic acid, dodecylbenzenesulphonic acid, dodecylsulphonic acid, hydrochloric acid, sulphuric acid or phosphoric acid.

22. Composition according to any one of the preceding claims, **characterized in that** the concave particles are formed (in longitudinal cross section) of a small internal arc (11), of a large external arc (21) and of segments (31) which connect the ends of the respective arcs, the width (W1) between the two ends of the small internal arc (11) ranging from 0.01 to 8 µm, preferably from 0.02 to 6 µm, on average, the width (W2) between the two ends of the large external arc (21) ranging from 0.05 to 10 µm, preferably from 0.06 to 8 µm, on average, and the height (H) of the large external arc (21) ranging from 0.015 to 8 µm, preferably from 0.03 to 6 µm, on average.

23. Composition according to any one of the preceding claims, **characterized in that** the concave particles are present in a content ranging from 0.01% to 50% by weight, with respect to the total weight of the composition, preferably ranging from 0.1% to 30% by weight and preferentially ranging from 1% to 15% by weight.

24. Composition according to any one of the preceding claims, **characterized in that** the particles with the shape of platelets are particles having a greatest dimension and a thickness such that the ratio of the greatest dimension to the thickness is greater than or equal to 5.

25. Composition according to any one of the preceding claims, **characterized in that** the platelets are chosen from platelets of inorganic materials.

26. Composition according to any one of the preceding claims, **characterized in that** the platelets are chosen from platelets formed of mica, sericite, glass, silica, aluminium oxide or barium sulphate.

27. Composition according to any one of the preceding claims, **characterized in that** the platelets are covered with a layer of metal or of metal oxide.

28. Composition according to the preceding claim, **characterized in that** the metal is chosen from silver, aluminium, chromium, nickel, molybdenum, gold, copper, tin, magnesium and their mixtures.

29. Composition according to Claim 27, **characterized in that** the metal oxide is chosen from titanium dioxide, iron oxides, zinc oxides, chromium oxide and their mixtures.

30. Composition according to Claim 27, **characterized in that** the metal oxide is titanium dioxide.

31. Composition according to any one of Claims 1 to 23, **characterized in that** the platelets are flat fibres.

32. Composition according to the preceding claim, **characterized in that** the flat fibres have a length L and a diameter D such that L/D is chosen within the range from 5 to 2500, preferably from 5 to 500 and better still from 5 to 150.

33. Composition according to Claim 31 or 32, **characterized in that** the fibres have a transverse cross section included within a circle with a diameter ranging from 2 nm to 500 µm, preferably ranging from 100 nm to 100 µm and better still from 1 µm to 70 µm.

34. Composition according to any one of Claims 31 to 33, **characterized in that** the flat fibres have a length L ranging from 1 µm to 10 mm, preferably from 0.1 mm to 5 mm and better still from 0.3 mm to 3.5 mm.

35. Composition according to any one of Claims 31 to 34, **characterized in that** the flat fibres have a transverse cross section exhibiting a greatest length L1 and a smallest length L2 such that L1/L2 is greater than 4, preferably greater than 7.

36. Composition according to the preceding claim, **characterized in that** L1/L2 ranges from 4 to 15, preferably from 6 to 12 and better still from 7 to 10.

37. Composition according to any one of Claims 31 to 36, **characterized in that** the flat fibres have a transverse cross section of rectangular, ovoid or ellipsoidal shape.

38. Composition according to any one of Claims 31 to 37, **characterized in that** the flat fibres are provided in the ribbon or tagliatelle form.

39. Composition according to any one of Claims 31 to 38, **characterized in that** the flat fibres have a count chosen within the range from 0.15 to 30 deniers and better still from 0.18 to 18 deniers.

40. Composition according to any one of Claims 31 to 39, **characterized in that** the flat fibres are fibres formed of polymers.

41. Composition according to any one of Claims 31 to 40, **characterized in that** the flat fibres are chosen from fibres formed of rayon, polyamide, viscose, acetate, in particular rayon acetate, poly(p-phenylene terephthalamide), acrylic polymer, in particular poly(methyl methacrylate) or poly(2-hydroxyethyl methacrylate), polyolefin and in particular polyethylene or polypropylene, polytetrafluoroethylene, poly(vinyl chloride) or poly(vinylidene chloride), poly(vinylidene fluoride), poly(vinyl alcohol), polyacrylonitrile, polyurethane, polyesters, such as poly(ethylene terephthalate)s or poly(ethylene naphthalate)s, or polycarbonate.

42. Composition according to any one of Claims 31 to 41, **characterized in that** the flat fibres are chosen from fibres with a multilayer structure comprising alternating layers of polymers chosen from polyesters, acrylic polymers and polyamides.

43. Composition according to any one of the preceding claims, **characterized in that** the particles with the shape of platelets are present in a content ranging from 1% to 99% by weight, with respect to the total weight of the composition, preferably ranging from 5% to 90% and preferentially ranging from 10% to 80% by weight.

44. Composition according to any one of the preceding claims, **characterized in that** the particles with the shape of platelets and the concave particles are present in a content such that the particles with the shape of platelets/concave particles ratio by weight ranges from 1 to 100, preferably ranges from 2 to 75 and preferentially ranges from 5 to 50.

45. Composition according to any one of the preceding claims, **characterized in that** the concave particles are present in a content ranging from 1% to 50% by weight, with respect to the total weight of the concave particles and of the particles with the shape of platelets, preferably ranging from 2% to 45% by weight and preferentially ranging from 5% to 40% by weight.

46. Composition according to any one of the preceding claims, **characterized in that** it comprises an additional pulverulent colouring material different from the said platelets and chosen from pigments and glitter.

47. Composition according to the preceding claim, **characterized in that** the pigments are chosen from titanium dioxide, zirconium oxide, cerium oxide, zinc oxides, iron oxides, chromium oxide, manganese violet, ultramarine blue, chromium hydrate, ferric blue, aluminium powder, copper powder, carbon black, pigments of D & C type, or lakes.

48. Composition according to Claim 46 or 47, **characterized in that** the pigments are present in a content ranging from 0.1% to 15% by weight, with respect to the total weight of the composition, preferably ranging from 0.5% to 12% by weight and preferentially ranging from 1% to 10% by weight.

49. Composition according to any one of the preceding claims, **characterized in that** it comprises an additional filler different from the said platelets.

50. Composition according to the preceding claim, **characterized in that** the additional filler is chosen from talc, mica, silica, kaolin, powders formed of polyamide, powders formed of poly-β-alanine, powders formed of polyethylene, powders formed of polyurethane, powders formed of tetrafluoroethylene polymers, lauryl-lysine, starch, boron nitride, polymeric hollow microspheres, silicone resin microbeads, particles formed of polyorganosiloxane elastomers, precipitated calcium carbonate, magnesium carbonate, basic magnesium carbonate, hydroxyapatite, hollow silica microspheres, glass or ceramic microcapsules, or metal soaps derived from organic carboxylic acids having from 8 to 22 carbon atoms.

51. Composition according to Claim 49 or 50, **characterized in that** the additional fillers are present in a content ranging from 0.1% to 90% by weight, with respect to the total weight of the composition, preferably ranging from 1% to 80% by weight and preferentially ranging from 5% to 70% by weight.

52. Composition according to any one of the preceding claims, **characterized in that** it comprises a liquid fatty phase.

53. Composition according to the preceding claim, **characterized in that** the liquid fatty phase comprises an oil chosen from mink oil, turtle oil, soybean oil, grape seed oil, sesame oil, maize oil, rapeseed oil, sunflower oil, cottonseed oil, avocado oil, olive oil, castor oil, jojoba oil, groundnut oil, liquid paraffins, squalane, liquid petrolatum, polydecene, isopropyl myristate, isopropyl palmitate, butyl stearate, isodecyl stearate, hexyl laurate, isononyl isononanoate, 2-ethylhexyl palmitate, 2-hexyldecyl laurate, 2-octyldecyl palmitate, 2-octyldodecyl myristate, 2-octyldodecyl lactate, di(2-ethylhexyl) succinate, diisostearyl malate, glyceryl triisostearate, diglyceryl triisostearate, silicone oils, fluorinated silicones, perfluorinated oils, oleic acid, linoleic acid, linolenic acid, cetanol or oleyl alcohol.

54. Composition according to Claim 52 or 53, **characterized in that** the liquid fatty phase is present in a content ranging from 0.1% to 13% by weight, with respect to the total weight of the composition, preferably ranging from 0.1% to 10% by weight and preferentially ranging from 0.1% to 8% by weight.

55. Composition according to any one of the preceding claims, **characterized in that** it comprises a cosmetic ingredient chosen from waxes, preservatives, cosmetic active principles, moisturizing agents, UV screening agents, thickeners, water, surfactants or fragrances.

56. Composition according to any one of the preceding claims, **characterized in that** it is provided in the form of a compact powder.

57. Composition according to any one of the preceding claims, **characterized in that** the composition is in the form of a blusher, an eyeshadow, a face powder, a foundation, a concealer, a product for making up the body, a product for caring for the face, a product for caring for the body or an antisun product.

58. Cosmetic process for making up or for the non-therapeutic treatment of keratinous substances, in particular the skin, comprising the application to the keratinous substances, in particular to the skin, of a composition according to any one of the preceding claims.

## Patentansprüche

1. Kosmetische Zusammensetzung, umfassend eine pulverförmige Phase, wobei die pulverförmige Phase konkave Partikel in Form von Abschnitten von Hohlkugeln und Partikel in Form von Plättchen umfasst, **dadurch gekennzeichnet, dass** die Partikel in Form von Abschnitten von Hohlkugeln aus einem siliciumorganischen Material bestehen.

2. Zusammensetzung nach dem vorhergehenden Anspruch, **dadurch gekennzeichnet, dass** die Partikel einen mittleren Durchmesser im Bereich zwischen 0,05 und 10 µm aufweisen.

3. Zusammensetzung nach Anspruch 1 oder 2, **dadurch gekennzeichnet, dass** die Partikel aus Abschnitten von Hohlkugeln einen hufeisenförmigen Querschnitt oder einen bogenförmigen Querschnitt aufweisen.

4. Zusammensetzung nach Anspruch 1, 2 oder 3, **dadurch gekennzeichnet, dass** das siliciumorganische Material ein vernetztes Polysiloxan mit dreidimensionaler Struktur ist, umfassend oder bestehend aus Einheiten mit der Formel (I): SiO₂ und mit der Formel (II): R¹SiO₁,₅,
wobei R¹ eine organische Gruppe bezeichnet, die ein Kohlenstoffatom aufweist, das direkt an das Siliciumatom gebunden ist.

5. Zusammensetzung nach dem vorhergehenden Anspruch, **dadurch gekennzeichnet, dass** die organische Gruppe eine reaktionsfähige organische Gruppe, eine nicht reaktionsfähige organische Gruppe und bevorzugt eine nicht reaktionsfähige organische Gruppe ist.

6. Zusammensetzung nach Anspruch 5, **dadurch gekennzeichnet, dass** die nicht reaktionsfähige organische Gruppe eine C₁-C₄-Alkylgruppe oder eine Phenylgruppe sein kann.

7. Zusammensetzung nach Anspruch 5 oder 6, **dadurch gekennzeichnet, dass** die nicht reaktionsfähige organische Gruppe eine Methylgruppe ist.

8. Zusammensetzung nach Anspruch 5, **dadurch gekennzeichnet, dass** die reaktionsfähige organische Gruppe aus einer Epoxygruppe, einer (Meth)acryloxygruppe, einer Alkenylgruppe, einer Mercaptoalkyl-, einer Aminoalkyl-, einer Halogenoalkylgruppe, einer Glyceroxygruppe, einer Ureidogruppe, einer Cyanogruppe ausgewählt ist.

9. Zusammensetzung nach Anspruch 5 oder 8, **dadurch gekennzeichnet, dass** die reaktionsfähige organische Gruppe aus einer Epoxygruppe, einer (Meth)acryloxygruppe, einer Alkenylgruppe, einer Mercaptoalkyl-, einer Aminoalkylgruppe ausgewählt ist.

10. Zusammensetzung nach Anspruch 4, **dadurch gekennzeichnet, dass** R¹ eine Methylgruppe bezeichnet.

11. Zusammensetzung nach einem der vorhergehenden Ansprüche, **dadurch gekennzeichnet, dass** das siliciumorganische Material die Einheiten (I) und (II) in einem Molverhältnis Einheit (I)/Einheit (II) im Bereich von 30/70 bis 50/50, bevorzugt im Bereich von 35/65 bis 45/55 umfasst.

12. Zusammensetzung nach einem der vorhergehenden Ansprüche, **dadurch gekennzeichnet, dass** die siliciumorganischen Partikel gemäß einem Verfahren erhalten werden können, umfassend:
(a) Einführen in ein wässriges Medium in Gegenwart mindestens eines Hydrolysekatalysators und gegebenenfalls mindestens eines grenzflächenaktiven Stoffs, einer Verbindung (III) mit der Formel SiX₄ und einer Verbindung (IV) mit der Formel RSiY₃, wobei X und Y unabhängig voneinander eine C₁-C₄-Alkoxygruppe, eine Alkoxyethoxygruppe, die eine C₁-C₄-Alkoxygruppe enthält, eine C₂-C₄-Acyloxygruppe, eine N,N-Dialkylaminogruppe, die eine C₁-C₄-Alkylgruppe enthält, eine Hydroxylgruppe, ein Halogenatom oder ein Wasserstoffatom bezeichnen, und R eine organische Gruppe bezeichnet, die ein Kohlenstoffatom umfasst, das direkt an das Siliciumatom gebunden ist; und
(b) Inkontaktbringen des aus Schritt (a) resultierenden Gemischs mit einer wässrigen Lösung, die mindestens einen Polymerisationskatalysator und gegebenenfalls mindestens einen grenzflächenaktiven Stoff enthält, bei einer Temperatur im Bereich zwischen 30 und 85 °C für mindestens zwei Stunden.

13. Zusammensetzung nach dem vorhergehenden Anspruch, **dadurch gekennzeichnet, dass** in Schritt (a) das Molverhältnis der Verbindung (III) zur Verbindung (IV) im Bereich von 30/70 bis 50/50, bevorzugt im Bereich von 35/65 bis 45/45 und vorzugsweise bei 40/60 liegt.

14. Zusammensetzung nach Anspruch 12 oder 13, **dadurch gekennzeichnet, dass** das Gewichtsverhältnis des Wassers zur Gesamtheit der Verbindungen (III) und (IV) in Schritt (a) im Bereich von 10/90 bis 70/30 liegt.

15. Zusammensetzung nach Anspruch 12, **dadurch gekennzeichnet, dass** die organische Gruppe eine reaktionsfähige organische Gruppe, eine nicht reaktionsfähige organische Gruppe und bevorzugt eine nicht reaktionsfähige organische Gruppe ist.

16. Zusammensetzung nach Anspruch 12, **dadurch gekennzeichnet, dass** die nicht reaktionsfähige organische Gruppe eine C₁-C₄-Alkylgruppe oder eine Phenylgruppe sein kann.

17. Zusammensetzung nach Anspruch 12 oder 16, **dadurch gekennzeichnet, dass** die nicht reaktionsfähige organische Gruppe eine Methylgruppe ist.

18. Zusammensetzung nach Anspruch 15, **dadurch gekennzeichnet, dass** die reaktionsfähige organische Gruppe aus einer Epoxygruppe, einer (Meth)acryloxygruppe, einer Alkenylgruppe, einer Mercaptoalkyl-, einer Aminoalkyl-, einer Halogenoalkylgruppe, einer Glyceroxygruppe, einer Ureidogruppe, einer Cyanogruppe ausgewählt ist.

19. Zusammensetzung nach Anspruch 15 oder 18, **dadurch gekennzeichnet, dass** die reaktionsfähige organische Gruppe aus einer Epoxygruppe, einer (Meth)acryloxygruppe, einer Alkenylgruppe, einer Mercaptoalkyl-, einer Aminoalkylgruppe ausgewählt ist.

20. Zusammensetzung nach Anspruch 12, **dadurch gekennzeichnet, dass** R eine Methylgruppe bezeichnet.

21. Zusammensetzung nach einem der Ansprüche 12 bis 20, **dadurch gekennzeichnet, dass** der Hydrolysekatalysator und der Polymerisationskatalysator unabhängig aus Natriumhydroxid, Kaliumhydroxid, Natriumcarbonat, Natriumhydrogencarbonat, Ammoniak, Trimethylamin, Triethylamin, Tetramethylammoniumhydroxid, Citronensäure, Essigsäure, Methansulfonsäure, p-Toluolsulfonsäure, Dodecylbenzolsulfonsäure, Dodecylsulfonsäure, Chlorwasserstoffsäure, Schwefelsäure, Phosphorsäure ausgewählt ist.

22. Zusammensetzung nach einem der vorhergehenden Ansprüche, **dadurch gekennzeichnet, dass** die konkaven Partikel (im Längsschnitt) aus einem inneren kleinen Bogen (11), einem äußeren großen Bogen (21) und Segmenten (31) gebildet werden, die jeweils die Enden der Bögen verbinden, wobei die Breite (W1) zwischen den beiden Enden des inneren kleinen Bogens (11) im Mittel im Bereich von 0,01 bis 8 µm, bevorzugt von 0,02 bis 6 µm liegt, die Breite (W2) zwischen den beiden Enden des äußeren großen Bogens (21) im Mittel im Bereich von 0,05 bis 10 µm, bevorzugt von 0,06 bis 8 µm liegt und die Höhe (H) des äußeren großen Bogens (21) im Mittel im Bereich von 0,015 bis 8 µm, bevorzugt von 0,03 bis 6 µm liegt.

23. Zusammensetzung nach einem der vorhergehenden Ansprüche, **dadurch gekennzeichnet, dass** die konkaven Partikel in einem Anteil im Bereich von 0,01 Gew.-% bis 50 Gew.-%, bezogen auf das Gesamtgewicht der Zusammensetzung, bevorzugt im Bereich von 0,1 Gew.-% bis 30 Gew.-%, und vorzugsweise im Bereich von 1 Gew.-% bis 15 Gew.-% vorhanden sind.

24. Zusammensetzung nach einem der vorhergehenden Ansprüche, **dadurch gekennzeichnet, dass** die Partikel in Form von Plättchen Partikel sind, die eine größere Abmessung und eine Dicke aufweisen, so dass das Verhältnis der größten Abmessung zur Dicke größer oder gleich 5 ist.

25. Zusammensetzung nach einem der vorhergehenden Ansprüche, **dadurch gekennzeichnet, dass** die Plättchen aus Plättchen aus mineralischen Materialien ausgewählt sind.

26. Zusammensetzung nach einem der vorhergehenden Ansprüche, **dadurch gekennzeichnet, dass** die Plättchen aus Glimmer-, Sericit-, Glas-, Siliciumdioxid-, Aluminiumoxid-, Bariumsulfatplättchen ausgewählt sind.

27. Zusammensetzung nach einem der vorhergehenden Ansprüche, **dadurch gekennzeichnet, dass** die Plättchen mit einer Metall- oder Metalloxidschicht bedeckt sind.

28. Zusammensetzung nach dem vorhergehenden Anspruch, **dadurch gekennzeichnet, dass** das Metall aus Silber, Aluminium, Chrom, Nickel, Molybdän, Gold, Kupfer, Zinn, Magnesium und deren Gemischen ausgewählt ist.

29. Zusammensetzung nach Anspruch 27, **dadurch gekennzeichnet, dass** das Metalloxid aus Titandioxid, Eisenoxiden, Zinkoxid, Chromoxid und deren Gemischen ausgewählt ist.

30. Zusammensetzung nach Anspruch 27, **dadurch gekennzeichnet, dass** das Metalloxid Titandioxid ist.

31. Zusammensetzung nach einem der Ansprüche 1 bis 23, **dadurch gekennzeichnet, dass** die Plättchen flache Fasern sind.

32. Zusammensetzung nach dem vorhergehenden Anspruch, **dadurch gekennzeichnet, dass** die flachen Fasern eine Länge L und einen Durchmesser D aufweisen, so dass L/D aus dem Bereich von 5 bis 2.500, bevorzugt von 5 bis 500 und besser von 5 bis 150 ausgewählt ist.

33. Zusammensetzung nach Anspruch 31 oder 32, **dadurch gekennzeichnet, dass** die Fasern einen Querschnitt, enthalten in einem Kreis mit einem Durchmesser im Bereich von 2 nm bis 500 µm, bevorzugt von 100 nm bis 100 µm und besser von 1 µm bis 70 µm aufweisen.

34. Zusammensetzung nach einem der Ansprüche 31 bis 33, **dadurch gekennzeichnet, dass** die flachen Fasern eine Länge L im Bereich von 1 µm bis 10 mm, bevorzugt von 0,1 mm bis 5 mm und besser von 0,3 mm bis 3,5 mm aufweisen.

35. Zusammensetzung nach einem der Ansprüche 31 bis 34, **dadurch gekennzeichnet, dass** die flachen Fasern einen Querschnitt aufweisen, der eine größere Länge L1 und eine kleinere Länge L2 aufweist, so dass L1/L2 größer als 4, bevorzugt größer als 7 ist.

36. Zusammensetzung nach dem vorhergehenden Anspruch, **dadurch gekennzeichnet, dass** L1/L2 im Bereich von 4 bis 15, bevorzugt von 6 bis 12 und besser von 7 bis 10 liegt.

37. Zusammensetzung nach einem der Ansprüche 31 bis 36, **dadurch gekennzeichnet, dass** die flachen Fasern einen rechteckigen, eiförmigen oder ellipsenförmigen Querschnitt aufweisen.

38. Zusammensetzung nach einem der Ansprüche 31 bis 37, **dadurch gekennzeichnet, dass** die flachen Fasern die Form eines Bandes oder von Tagliatelle aufweisen.

39. Zusammensetzung nach einem der Ansprüche 31 bis 38, **dadurch gekennzeichnet, dass** die flachen Fasern einen Titer aufweisen, der aus dem Bereich von 0,15 bis 30 Denier und besser 0,18 bis 18 Denier ausgewählt ist.

40. Zusammensetzung nach einem der Ansprüche 31 bis 39, **dadurch gekennzeichnet, dass** die flachen Fasern Polymerfasern sind.

41. Zusammensetzung nach einem der Ansprüche 31 bis 40, **dadurch gekennzeichnet, dass** die flachen Fasern aus Rayonfasern, Polyamidfasern, Viskosefasern, Acetatfasern, insbesondere Rayonacetatfasern, Poly-(p-phenylenterephthalamid)fasern, Acrylpolymerfasern, insbesondere Polymethylmethacrylatfasern oder Poly-2-hydroxyethylmethacrylatfasern, Polyolefinfasern und insbesondere Polyethylenfasern oder Polypropylenfasern, Polytetrafluorethylenfasern, Polyvinylchloridfasern oder Polyvinylidenchloridfasern, Polyvinylidenfluoridfasern,
Polyvinylalkoholfasern, Polyacrylnitrilfasern, Polyurethanfasern, Polyesterfasern, wie Polyethylenterephthalatfasern, Polyethylennaphthalatfasern, Polycarbonatfasern ausgewählt sind.

42. Zusammensetzung nach einem der Ansprüche 31 bis 41, **dadurch gekennzeichnet, dass** die flachen Fasern aus den Fasern mit Mehrschichtstruktur mit alternierenden Polymerschichten, ausgewählt aus Polyestern, Acrylpolymeren, Polyamiden, ausgewählt sind.

43. Zusammensetzung nach einem der vorhergehenden Ansprüche, **dadurch gekennzeichnet, dass** die Partikel in Form von Plättchen in einem Anteil im Bereich von 1 bis 99 Gew.-%, bezogen auf das Gesamtgewicht der Zusammensetzung, bevorzugt im Bereich von 5 Gew.-% bis 90 Gew.-% und vorzugsweise im Bereich von 10 Gew.-% bis 80 Gew.-% vorhanden sind.

44. Zusammensetzung nach einem der vorhergehenden Ansprüche, **dadurch gekennzeichnet, dass** die Partikel in Form von Plättchen und die konkaven Partikel in einem Anteil vorhanden sind, so dass das Gewichtsverhältnis von Partikeln mit Plättchenform/konkaven Partikeln im Bereich von 1 bis 100, bevorzugt im Bereich von 2 bis 75 und vorzugsweise im Bereich von 5 bis 50 liegt.

45. Zusammensetzung nach einem der vorhergehenden Ansprüche, **dadurch gekennzeichnet, dass** die konkaven Partikel in einem Anteil vorhanden sind, der im Bereich von 1 Gew.-% bis 50 Gew.-%, bezogen auf das Gesamtgewicht der konkaven Partikel und der Partikel in Form von Plättchen, bevorzugt im Bereich von 2 Gew.-% bis 45 Gew.-% und vorzugsweise im Bereich von 5 bis 40 Gew.-% liegt.

46. Zusammensetzung nach einem der vorhergehenden Ansprüche, **dadurch gekennzeichnet, dass** sie einen zusätzlichen pulverförmigen Farbstoff umfasst, der von den Plättchen verschieden und aus Pigmenten und Flitterchen ausgewählt ist.

47. Zusammensetzung nach dem vorhergehenden Anspruch, **dadurch gekennzeichnet, dass** die Pigmente aus Titandioxid, Zirconiumoxid, Ceroxid, Zinkoxiden, Eisenoxiden, Chromoxid, Manganviolett, Ultramarinblau, Chromhydrat, Eisenblau, Aluminiumpulver, Kupferpulver, Kohleschwarz, D&C-Pigmenten, Lacken ausgewählt sind.

48. Zusammensetzung nach Anspruch 46 oder 47, **dadurch gekennzeichnet, dass** die Pigmente in einem Anteil im Bereich von 0,1 Gew.-% bis 15 Gew.-%, bezogen auf das Gesamtgewicht der Zusammensetzung, bevorzugt im Bereich von 0,5 Gew.-% bis 12 Gew.-% und vorzugsweise im Bereich von 1 Gew.-% bis 10 Gew.-% vorhanden sind.

49. Zusammensetzung nach einem der vorhergehenden Ansprüche, **dadurch gekennzeichnet, dass** sie einen zusätzlichen Füllstoff umfasst, der verschieden von den Plättchen ist.

50. Zusammensetzung nach dem vorhergehenden Anspruch, **dadurch gekennzeichnet, dass** der zusätzliche Füllstoff aus Talkum, Glimmer, Siliciumdioxid, Kaolin, Polyamidpulvern, Poly-beta-alaninpulvern, Polyethylenpulvern, Polyurethanpulvern, Tetrafluorethylenpolymerpulvern, Lauroyllysin, Stärke, Bornitrid, Polymermikrohohlkugeln, Siliconharzmikrokügelchen, elastomeren Polyorganosiloxanpartikeln, gefälltem Calciumcarbonat, Magnesiumcarbonat und Magnesiumhydrocarbonat, Hydroxyapatit, Siliciumdioxid-Mikrohohlkugeln, Mikrokapseln aus Glas oder Keramik, von organischen Carbonsäuren mit 8 bis 22 Kohlenstoffatomen abgeleiteten Metallseifen ausgewählt ist.

51. Zusammensetzung nach Anspruch 49 oder 50, **dadurch gekennzeichnet, dass** die zusätzlichen Füllstoffe in einem Anteil im Bereich von 0,1 Gew.-% bis 90 Gew.-%, bezogen auf das Gesamtgewicht der Zusammensetzung, bevorzugt im Bereich von 1 Gew.-% bis 80 Gew.-% und vorzugsweise im Bereich von 5 Gew.-% bis 70 Gew.-% vorhanden sind.

52. Zusammensetzung nach einem der vorhergehenden Ansprüche, **dadurch gekennzeichnet, dass** sie eine flüssige Fettphase umfasst.

53. Zusammensetzung nach dem vorhergehenden Anspruch, **dadurch gekennzeichnet, dass** die flüssige Fettphase ein Öl umfasst, das aus Nerzöl, Schildkrötenöl, Sojaöl, Traubenkernöl, Sesamöl, Maisöl, Rapsöl, Sonnenblumenöl, Baumwollsamenöl, Avocadoöl, Olivenöl, Ricinusöl, Jojobaöl, Erdnussöl, Paraffinölen, Squalan, Vaseline, Polydecen, Isopropylmyristat, Isopropylpalmitat, Butylstearat, Isodecylstearat, Hexyllaurat, Isononylisononanoat, 2-Ethylhexylpalmitat, 2-Hexyldecyllaurat, 2-Octyldecylpalmitat, 2-Octyldodecylmyristat oder -lactat, 2-Diethylhexylsuccinat, Diisostearylmalat, Glycerin- oder Diglycerintriisostearat, Siliconölen, fluorierten Siliconen, perfluorierten Ölen, Ölsäure, Linolsäure, Linolensäure, Cetanol, Oleylalkohol ausgewählt ist.

54. Zusammensetzung nach Anspruch 52 oder 53, **dadurch gekennzeichnet, dass** die flüssige Fettphase in einem Anteil im Bereich von 0,1 Gew.-% bis 13 Gew.-%, bezogen auf das Gesamtgewicht der Zusammensetzung, bevorzugt im Bereich von 0,1 Gew.-% bis 10 Gew.-% und vorzugsweise im Bereich von 0,1 Gew.-% bis 8 Gew.-% vorhanden ist.

55. Zusammensetzung nach einem der vorhergehenden Ansprüche, **dadurch gekennzeichnet, dass** sie einen kosmetischen Bestandteil umfasst, der aus Wachsen, Konservierungsmitteln, kosmetischen Wirkstoffen, Hydratisierungsmitteln, UV-Filtern, Verdickungsmitteln, Wasser, grenzflächenaktiven Stoffen und Parfums ausgewählt ist.

56. Zusammensetzung nach einem der vorhergehenden Ansprüche, **dadurch gekennzeichnet, dass** sie in Form von Kompaktpuder vorliegt.

57. Zusammensetzung nach einem der vorhergehenden Ansprüche, **dadurch gekennzeichnet, dass** die Zusammensetzung in Form von Wangenrouge, Lidschatten, Gesichtspuder, Make-up, eines Produkts gegen Augenringe, eines Produkts zum Schminken des Körpers, eines Produkts zur Gesichtspflege, eines Produkts zur Körperpflege und eines Sonnenschutzprodukts vorliegt.

58. Kosmetisches Verfahren zum Schminken oder zur nichttherapeutischen Behandlung von Keratinsubstanzen, insbesondere der Haut, umfassend das Aufbringen auf die Keratinsubstanzen, insbesondere auf die Haut, einer Zusammensetzung nach einem der vorhergehenden Ansprüche.
